# EUROPEAN PATENT SPECIFICATION

(11) **EP 2 773 293 B1**
(45) Date of publication and mention of the grant of the patent: **30.08.2017**
(21) Application number: 12846553.1
(22) Date of filing: 05.11.2012
(51) Int. Cl.: A61F 2/44, A61B 17/70, A61F 2/28, A61F 2/30

(54) **IMPLANT FOR LENGTH PRESERVATION DURING BONE REPAIR**
IMPLANTAT ZUR LÄNGENBEIBEHALTUNG BEI EINER KNOCHENREPARATUR
IMPLANT POUR PRÉSERVATION DE LA LONGUEUR AU COURS D'UNE RÉPARATION OSSEUSE

(30) Priority: 03.11.2011 US 201161555360 P
(43) Date of publication of application: 10.09.2014
(73) Proprietor: 4-web, Inc., Frisco, TX 75033 (US)
(72) Inventor: HUNT, Jessee, Plano, Texas 75024 (US); CRATES, John, M., Plano, Texas 75093 (US)
(74) Representative: Awapatent AB
(86) International application number: PCT/US2012/063600
(87) International publication number: WO 2013/067528

(56) References cited:
- WO-A1-01/28460
- WO-A1-2010/080511
- WO-A2-2008/022206
- US-A1- 2004 082 999
- US-A1- 2007 027 544
- US-A1- 2010 161 061
- US-B1- 6 931 812
- MURR, L, E. ET AL. ET AL.: 'Next-generation biomedical implants using additive manufacuring of complex, cellular and functional mesh arrays' PHILOSOPHICAL TRANSACTIONS OF THE ROYAL SOCIETY vol. 368, 22 March 2010, pages 1999 - 2032, XP055149555

## Description

### BACKGROUND

### 1. Field of the Invention

The present invention relates generally to medical devices and, more specifically, to implants.

### 2. Description of the Related Art

Implants may be used in human and/or animals to support and/or secure one or more bones. For example, implants may be used in the spine to support and/or replace damaged tissue between the vertebrae in the spine. Once implanted between two vertebrae, the implant may provide support between the two vertebrae and bone growth may take place around and through the implant to at least partially fuse the two vertebrae for long-term support. Implants may include relatively large rims with solid material that may cover, for example, 50% of the area that interacts with the endplate. The rim may provide a contact area between the implant and the vertebral endplates. Large rims may have several drawbacks. For example, large rims may impede bone growth and reduce the size of the bone column fusing the superior and inferior vertebral bodies.

Spinal implants may include open channels through the center of the supporting rims in a superior/inferior direction. The open channel design may require members of the implant that separate the rims that interact with the vertebral endplates to absorb the compressive forces between the vertebral endplates. This may increase the pressure on smaller areas of the vertebral endplates and may potentially lead to stress risers in the vertebral endplates. Further, while bone graft material is often used in conjunction with implants to encourage bone growth, the open column design of implants may reduce the likelihood of bone graft material from securing itself to the implant which could result in a bio-mechanical cooperation that is not conducive to promoting good fusion.

Bone graft material may be packed into the implant in a high-pressure state to prevent bone graft material from exiting the implant while being placed between the vertebral endplates. The high-pressure state may also reduce the potential for the bone graft material loosening due to motion between the implant and the vertebral endplates or compressive forces experienced during settling of the implant. In addition, a high-pressure environment may allow the bone graft material to re-model and fuse at greater strength. High-pressure states, however, may be difficult to create and maintain for the bone graft material in an implant.

The closest prior art is disclosed by US 2010/0161061 A1.

### SUMMARY

Various embodiments of implant systems and related apparatus, and methods of operating the same are described herein. In various embodiments, provided is an implant for interfacing with a bone structure includes a web structure, including a space truss, configured to interface with human bone tissue. The space truss includes two or more planar truss units having a plurality of struts joined at nodes.

In certain embodiments, an implant includes a web structure configured to interface with human bone tissue. The implant includes a space truss and an external truss. The space truss includes two or more planar truss units having a plurality of struts joined at nodes. The external truss includes one or more planar trusses having two or more adjacent planar truss units that lie in substantially the same plane.

In some embodiments, the planar truss units include a planar triangular truss unit having three substantially straight struts and three nodes in a triangular configuration. The space truss may include a plurality of planar truss units coupled to one another, wherein each of the truss units lies in a plane that is not substantially parallel to a plane of an adjacent truss unit that shares at least one strut.

In some embodiments, at least one strut passes through the central portion of the implant. At least one strut may connect two or more opposing vertices of the square shaped common truss unit. At least one strut may connect two opposed vertices of the octahedron.

In one embodiment, an implant is composed of a web structure that includes a plurality of planar truss units coupled to each other, the planar truss units comprising a plurality of struts coupled to a plurality of nodes, wherein one or more angles defined by two struts and a node of one or more planar truss units are different than one or more corresponding angles defined by two struts and a node of one or more other planar truss units, wherein connecting exterior surface struts couple the nodes of the non-equivalent angle planar truss units to each other, defining a non-planar surface.

In an embodiment, the plurality of planar truss units comprise one or more planar triangular truss units having three substantially straight struts and three nodes in a triangular configuration. The plurality of planar truss units may be coupled to one another such that one or more planar truss units lie in a plane that is not substantially parallel to a plane of a planar truss unit that shares at least one strut with the one or more planar truss units. In some embodiments, a plurality of planar truss units defines an exterior surface of the web structure. The plurality of planar truss units may include a first planar triangular truss unit coupled to a second planar triangular truss unit, wherein the first and second planar triangular truss units are coupled in an opposing manner with a single node defining the apex of each planar triangular truss unit.

In an embodiment, at least some of the connecting struts define triangular trusses having at least one node shared by two different triangular planar truss units having different corresponding angles. The connecting struts may define a non-planar contact surface of the implant. In some embodiments, the non-planar surface is a substantially rounded surface. Opposing surfaces of the implant may be non-planar and/or rounded. In some embodiments, ne or more of the connecting exterior surface struts are curved to form a rounded exterior surface of the implant.

In another embodiment, an implant is composed of a web structure, wherein the web structure includes a plurality of polar struts extending from one surface of the implant to the opposing surface of the implant; and a plurality of connecting struts, coupled to the ends of the polar struts, together defining a plurality of planar truss units, wherein at least two of the polar struts have different lengths such that the web structure has at least one non-planar surface defined by the ends of the polar struts.

In an embodiment, the plurality of planar truss units comprise one or more planar triangular truss units having three substantially straight struts and three nodes in a triangular configuration. The plurality of planar truss units may be coupled to one another such that one or more planar truss units lie in a plane that is not substantially parallel to a plane of a planar truss unit that shares at least one strut with the one or more planar truss units. In some embodiments, a plurality of planar truss units defines an exterior surface of the web structure. The exterior surface of the web structure defined by the plurality of planar truss units is substantially parallel to the longitudinal axis of the polar struts. The plurality of planar truss units may include a first planar triangular truss unit coupled to a second planar triangular truss unit, wherein the first and second planar triangular truss units are coupled in an opposing manner with a single node defining the apex of each planar triangular truss unit.

In some embodiments, at least some of the connecting struts define triangular trusses having at least one node at the end of a polar strut. The non-planar surface may have a substantially rounded surface. In some embodiments, opposing surfaces of the implant are rounded. At least a portion of the struts coupled to a node at the end of a polar strut may be curved to form a rounded exterior surface of the implant.

In an embodiment, an implant comprises a web structure, the web structure comprising: a plurality of planar truss units coupled to each other, the planar truss units comprising a plurality of struts coupled to a plurality of nodes; wherein one or more angles defined by two struts and a node of one or more planar truss units are different than one or more corresponding angles defined by two struts and a node of one or more other planar truss units, wherein connecting exterior surface struts couple the nodes of the non-equivalent angle planar truss units to each other, defining a non-planar surface. The implant may be made by a method that includes applying a layer of material to a support, converting the applied material into a solid, wherein the solid material forms a portion of one or more planar truss units; and removing the implant from the support.

In one embodiment, converting the applied layer into a solid comprises moving a directed energy source relative to the support to melt a portion of the material, wherein the directed energy source is moved in a pattern determined from a three-dimensional model of at least a portion of the web structure.

In an embodiment, a method of length preservation in a separated bone includes forming a contact surface on a first side of the separated bone; forming a contact surface on a second side of the separated bone; placing an implant in contact with the first and second contact surfaces of the separated bone; wherein the implant comprises: a web structure comprising a space truss comprising two or more planar truss units having a plurality of struts joined at nodes. In an embodiment, the implant comprises opposing non-planar surfaces. In an embodiment, the method also includes forming a groove in one of the contact surfaces of the separated bone. The method further includes aligning the implant with a groove formed in one of the contact surfaces of the separated bone. In some embodiments the method includes attaching a coupling member to the separated bone such that the coupling member couples the first side of the separated bone with the second side of the separated bone.

In an embodiment, the implant used in the length preservation process includes a plurality of polar struts extending from one surface of the implant to the opposing surface of the implant; and a plurality of connecting struts, coupled to the ends of the polar struts, together defining a plurality of planar truss units, wherein at least two of the polar struts have different lengths defining the opposing non-planar surfaces. In another embodiment, the implant used in the length preservation process includes a plurality of planar truss units coupled to each other, the planar truss units comprising a plurality of struts coupled to a plurality of nodes; wherein one or more angles defined by two struts and a node of one or more planar truss units are different than one or more corresponding angles defined by two struts and a node of one or more other planar truss units, wherein the nodes of the non-equivalent angle planar truss units are coupled to each other with one or more exterior struts, the exterior struts defining a non-planar surface.

In an embodiment, a method of length preservation in a first metatarsal phalangeal arthrodesis includes: reaming the metatarsal side of the joint such that a non-planar surface is formed on the metatarsal side of the joint; reaming the phalangeal side of the joint such that a non-planar surface is formed on the phalangeal side of the joint; placing an implant in contact with the non-planar surface of the metatarsal side of the joint and the non-planar surface of the phalangeal side of the joint; wherein the implant comprises: a web structure comprising a space truss comprising two or more planar truss units having a plurality of struts joined at nodes, wherein the implant comprises opposing non-planar surfaces; wherein the non-planar surface of the phalangeal side of the joint is substantially complementary with a first non-planar surface of the implant; and wherein the non-planar surface of the metatarsal side of the joint is substantially complementary with a second non-planar surface of the implant, opposite the first non-planar surface of the implant.

In an embodiment, an implant is composed of a web structure, wherein the web structure includes a plurality of side surface planar truss units defining an exterior side surface of the implant, the side surface planar truss units comprising a plurality of struts coupled to a plurality of nodes; a plurality of surface struts extending from one or more of the nodes of one or more of the side surface planar truss units toward the interior portion of the implant, wherein the angle between the surface struts and the struts defining the side surface planar truss units is substantially greater than, or substantially less than 90 ; and a plurality of interior nodes coupling the surface struts to each other to define an exterior surface of the implant.

In an embodiment, an implant is composed of a web structure that includes a space truss comprising two or more planar truss units having a plurality of struts joined at nodes, wherein the implant has at least one non-planar surface.

In an embodiment, a method includes installing, between two separated bone portions, a web structure comprising a space truss comprising two or more planar truss units having a plurality of struts joined at nodes.

### BRIEF DESCRIPTION OF THE DRAWINGS

A better understanding of the present invention may be obtained when the following detailed description is considered in conjunction with the following drawings, in which:
FIGS. 1A-1B illustrate views of an implant with lordosis, according to an embodiment;
FIGS. 2A-2D illustrate views of an implant without lordosis, according to an embodiment;
FIGS. 3A-3C illustrate progressive sectioned views of the implant showing the internal structure of the implant, according to an embodiment;
FIG. 3D illustrates an isometric view of the implant, according to an embodiment;
FIGS. 4A-4D illustrate another configuration of the web structure, according to an embodiment;
FIG. 5 illustrates a flowchart of a method for making an implant, according to an embodiment;
FIGS. 6A-6C depict an embodiment of an implant having non-planar surfaces;
FIGS. 7A and 7B depict an embodiment of an implant having a convex upper surface and a concave lower surface;
FIGS. 8A and 8B depict an embodiment of an alternate implant having a convex upper surface and a concave lower surface;
FIGS. 9A-9E depict perspective views of implants positioned between bone portions to fuse the bone portions;
FIG. 10 depicts a perspective view of an alternate embodiment of a tapered implant;
FIG. 11 depicts an alternative embodiment of an Evan's wedge; and
FIG. 12 depicts an alternate embodiment of an implant having a non-planar surface.
FIG. 13 illustrates a truss structure disposed on an implant, according to an embodiment;

While the invention is susceptible to various modifications and alternative forms, specific embodiments thereof are shown by way of example in the drawings and will herein be described in detail. It should be understood, however, that the drawings and detailed description thereto are not intended to limit the invention to the particular form disclosed, but on the contrary, the intention is to cover all modifications, equivalents, and alternatives falling within the scope of the present invention as defined by the appended claims. Note, the headings are for organizational purposes only and are not meant to be used to limit or interpret the description or claims.

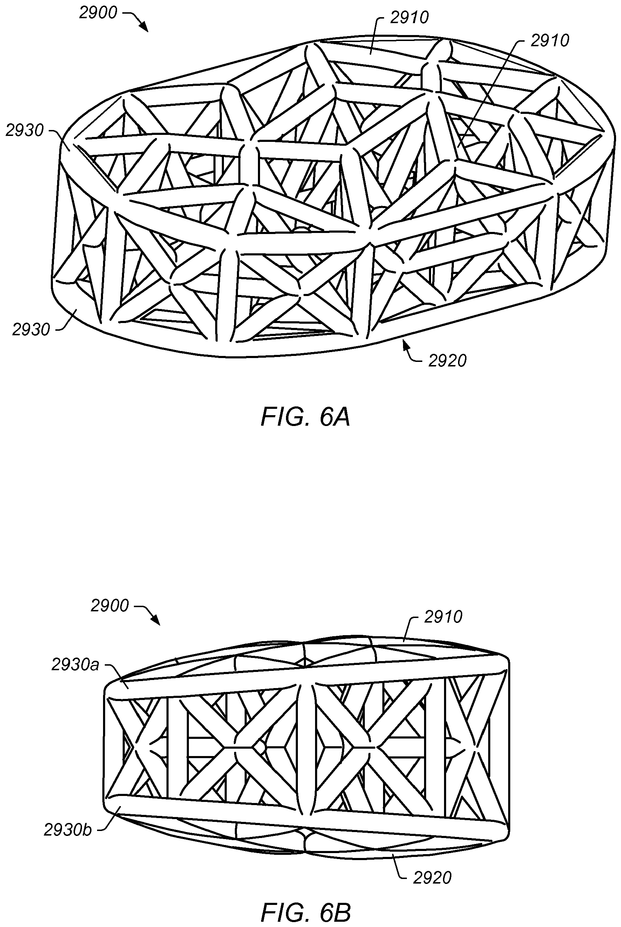

The invention is particularly described with reference to figures 6a-6c.

### DETAILED DESCRIPTION OF THE EMBODIMENTS

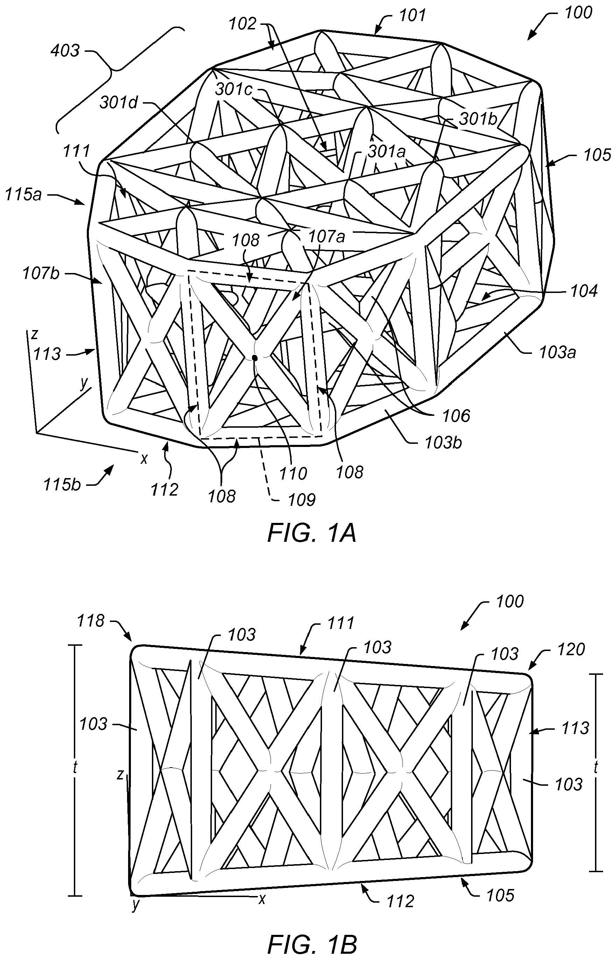

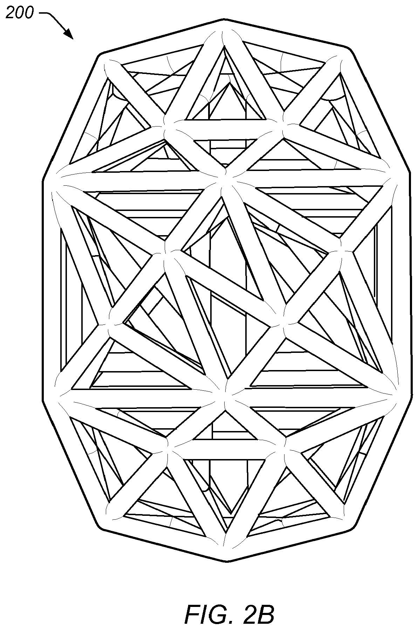

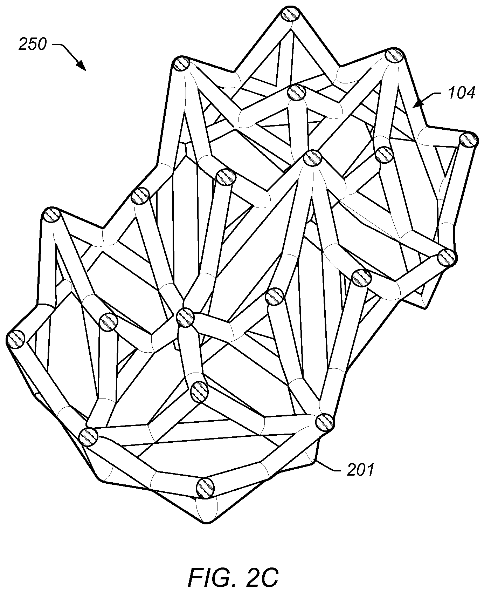

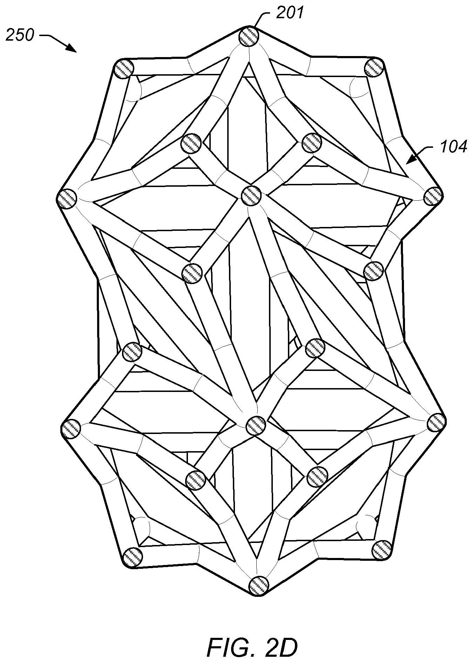

FIGS. 1A-1B illustrate views of implant 100, according to an embodiment. Implant 100 may be used, for example, in anterior lumbar inter-body fusion (ALIF) or posterior lumbar inter-body fusion (PLIF). In some embodiments, implant 100 may include a web structure 101 with one or more trusses 102 (e.g., planar and space trusses). Implant 100 and its web structure 101 may be used in various types of implants for humans or animals such as spinal implants (e.g., see FIGS. 1A-2D and 3A-4D)) and corpectomy devices (e.g., see FIGS. 2C-2D). Other implant uses are also contemplated.

As used herein a "truss" is a structure having one or more elongate struts connected at joints referred to as nodes. Trusses may include variants of a pratt truss, king post truss, queen post truss, town's lattice truss, planar truss, space truss, and/or a vierendeel truss (other trusses may also be used). Each unit (e.g., region having a perimeter defined by the elongate struts) may be referred to as a "truss unit."

As used herein a "planar truss" is a truss structure where all of the struts and nodes lie substantially within a single two-dimensional plane. A planar truss, for example, may include one or more "truss units" where each of the struts is a substantially straight member such that the entirety of the struts and the nodes of the one or more truss units lie in substantially the same plane. A truss unit where each of the struts is a substantially straight member such that the entirety of the struts and the nodes of the truss units lie in substantially the same plane is referred to as a "planar truss unit."

As used herein a "space truss" is a truss having struts and nodes that are not substantially confined in a single two-dimensional plane. A space truss may include two or more planar trusses (e.g., planar truss units) wherein at least one of the two or more planar trusses lies in a plane that is not substantially parallel to a plane of at least one or more of the other two or more planar trusses. A space truss, for example, may include two planar truss units adjacent to one another (e.g., sharing a common strut) wherein each of the planar truss units lie in separate planes that are angled with respect to one another (e.g., not parallel to one another).

As used herein a "triangular truss" is a structure having one or more triangular units that are formed by three straight struts connected at joints referred to as nodes. For example, a triangular truss may include three straight elongate strut members that are coupled to one another at three nodes to from a triangular shaped truss. As used herein a "planar triangular truss" is a triangular truss structure where all of the struts and nodes lie substantially within a single two-dimensional plane. Each triangular unit may be referred to as a "triangular truss unit." A triangular truss unit where each of the struts is a substantially straight member such that the entirety of the struts and the nodes of the triangular truss units lie in substantially the same plane is referred to as a "planar triangular truss unit." As used herein a "triangular space truss" is a space truss including one or more triangular truss units.

As used herein a "quadrilateral truss" is a structure having one or more quadrilateral units that are formed by four straight struts connected at joints referred to as nodes. For example, a quadrilateral truss may include four straight elongate strut members that are coupled to four nodes to from a quadrilateral shaped truss. As used herein a "planar quadrilateral truss" is a quadrilateral truss structure where all of the struts and nodes lie substantially within a single two-dimensional plane. Each quadrilateral unit may be referred to as a "quadrilateral truss unit." A quadrilateral truss unit where each of the struts is a substantially straight member such that the entirety of the struts and the nodes of the triangular truss units lie in substantially the same plane is referred to as a "planar quadrilateral truss unit." As used herein a "quadrilateral space truss" is a space truss including one or more quadrilateral truss units.

In various embodiments, the trusses 102 of web structure 101 may include one or more planar truss units (e.g., planar triangular truss units) constructed with straight or curved/arched members (e.g., struts) connected at various nodes. In some embodiments, the trusses 102 may be micro-trusses. A "micro-truss" may include a truss having dimensions sufficiently small enough such that a plurality of micro-trusses can be assembled or other wise coupled to one another to form a web structure having a small enough overall dimension (e.g., height, length and width) such that substantially all of the web structure can be inserted into an implant location (e.g., between two vertebra). Such a web structure and its micro-trusses can thus be employed to receive and distribute throughout the web structure loading forces of the surrounding tissue (e.g., vertebra, bone, or the like). In one embodiment, the diameters of the struts forming the micro-truss may be between about 0.25 millimeters (mm) and 5mm in diameter (e.g., a diameter of about 0.25mm, 0.5 mm, 0.6 mm, 0.7 mm, 0.8 mm, 0.9 mm, 1 mm, 2 mm, 3 mm, 4 mm, or 5 mm). In one embodiment, a micro-truss may have an overall length or width of less than about 1 inch (e.g., a length less than about 0.9 in, 0.8 in, 0.7 in, 0.6 in, 0.5 in, 0.4 in, 0.3 in, 0.2 in, 0.1 in).

As depicted, for example, in FIGS. 1A-1B, web structure 101 may extend throughout implant 100 (including the central portion of implant 100) to provide support throughout implant 100. Trusses 102 of implant 100 may thus support implant 100 against tensile, compressive, and shear forces. The web structure of trusses 102 may also reinforce implant 100 along multiple planes. The external truss structure may, for example, provide support against tensile and compressive forces acting vertically through the implant, and the internal web structure may provide support against tensile, compressive, and shear forces along the various planes containing the respective trusses. In some embodiments, the web structure includes trusses 102 that form a triangulated web structure with multiple struts (e.g., struts 103a-f) (struts are generally referred to herein as "struts 103").

In one embodiment, web structure 101 of the implant 100 may include an internal web structure that is at least partially enclosed by an external truss structure. For example, in one embodiment, web structure 101 may include an internal web structure that includes a space truss having at least a portion of the space truss surrounded by an external truss structure that includes one or more planar trusses formed with a plurality of planar truss units that lie substantially in a single plane. FIG. 1A depicts an embodiment of implant 100 and web structure 101 that includes internal web structure 104 and an external truss structure 105. In the illustrated embodiment, internal web structure 104 includes a space truss defined by a plurality of planar truss units 106 coupled at an angle with respect to one another such that each adjacent truss unit is not co-planar with each adjacent truss units. Adjacent truss units may include two truss units that share a strut and the respective two nodes at the ends of the shared strut.

In one embodiment, external truss structure 105 includes a plurality of planar trusses that are coupled about an exterior, interior or other portion of the implant. For example, in the illustrated embodiment, the external truss structure 105 includes a series of planar trusses 107a,b that are coupled to one another. Each planar truss 107a,b includes a plurality of planar truss units 108 that are coupled to one another and lie substantially in the same plane. As depicted, planar truss 107a includes four triangular planar truss units 108 having a common vertex 110 and arranged to form a generally rectangular structure that lies in a single common plane 109. In other words, the four truss units are arranged to form a substantially rectangular structure having "X" shaped struts extend from one corner of the rectangular structure to the opposite corner of the rectangular structure. As depicted, the substantially rectangular structure may include a trapezoidal shape. As described in more detail below, the trapezoidal shape may be conducive to providing an implant including lordosis. Lordosis may include an angled orientation of surfaces (e.g., top and bottom) of an implant that provides for differences in thickness in anterior and posterior regions of the implant such that the implant is conducive for supporting the curvature of a vertebral column.

In one embodiment, the planar trusses that form the external truss are coupled to one another, and are aligned along at least one axis. For example, in FIG. 1A, planar truss section 107a is coupled to an adjacent planar truss 107b. Planer truss sections 107a,b are not parallel in all directions. Planar truss sections 107a,b are, however, arranged parallel to one another in at least one direction (e.g., the vertical direction between the top and the bottom faces of implant 100). For example, planar trusses 107a,b and the additional planar trusses are arranged in series with an angle relative to one another to form a generally circular or polygon shaped enclosure having substantially vertical walls defined by the planar trusses and the planar truss units arranged in the vertical direction.

In one embodiment, the external truss portion may encompass the sides, top, and/or bottom of the implant. For example, in one embodiment, the external truss portion may include a top region, side regions, and/or a bottom region. FIG. 1A depicts an embodiment of implant 100 wherein external truss portion 105 includes a top 111, bottom 112 and a side region 113. As described above, side region 113 includes a series of planar trusses arranged vertically to form a circular/polygon ring-like structure that completely or at least partially surrounds the perimeter of the space truss disposed in the central portion of implant 100. In the depicted embodiment, top portion 111 of external truss structure 105 includes a plurality of truss units coupled to one another to form a planar truss that cover substantially the entire top region of internal web structure 104. In the illustrated embodiment, the top portion 111 spans entirely the region between top edges of the side portion 113 of external truss structure 105. In the illustrated embodiment, top portion 111 is formed from a single planar truss that includes a plurality of truss units that lie in substantially the same plane. In other words, the planar truss of top portion 111 defines a generally flat surface. Although difficult to view in FIG. 1, the underside of implant 100 may include the bottom portion 112 having a configuration similar to that of the top portion 111. In other embodiments, external truss structure 105 may include a partial side, top and/or bottom external truss portions. Or may not include one or more of the side, top and bottom external truss portions. For example, as described in more detail below, FIG. 2C depicts an embodiment of implant 100 than includes an internal web structure 104 that includes a space truss, and does not have an external truss structure.

In some embodiments, implant 100 may be composed of a biocompatible material such as a titanium alloy (e.g., γTitanium Aluminides), cobalt, chromium, stainless steel, Polyetheretherketone (PEEK), ceramics, etc. Other materials are also contemplated. For example, biodegradable polymers (e.g., poly(lactic-co-glycolic acid) or synthetic bone materials (e.g., hydroxyapatite) may be used. In some embodiments, implant 100 may be made through a rapid prototyping process (e.g., electron beam melting (EBM) process) as further described below. Other processes are also possible (e.g., injection molding, casting, sintering, selective laser sintering (SLS), Direct Metal Laser Sintering (DMLS), etc). SLS may include laser-sintering of high-performance polymers such as that provided by EOS of North America, Inc., headquartered in Novi, Michigan, U.S.A. High-performance polymers may include various forms of PEEK (e.g., HP3 having a tensile strength of up to about 95 mega Pascal (MPa) and a Young's modulus of up to about 4400 MPa and continuous operating temperature between about 180 °C (356 °F) and 260 °C (500 °F)). Other materials may include PA 12 and PA 11 provided by EOS of North America, Inc.

As depicted in FIG. 1B, implant 100 may include lordosis (e.g., an angle in top and/or bottom surfaces 115a,b approximately in a range of 4 to 15 degrees (such as 4, 5, 6, 7, 8, 9, 10, 11, 12, 13, 14, or 15 degrees)) to further support the adjacent vertebrae when implanted. As described above, lordosis may include an angled orientation of surfaces (e.g., top and bottom) that provide for differences in thickness in the anterior and posterior portions of the implant such that the implant is conducive for supporting the curvature of a vertebral column. In the illustrated embodiment, the thickness of implant 100 is greater at or near the anterior portion 118 and lesser at or near the posterior portion 120 of the implant. In the illustrated embodiment, the side portions of external truss structure 105 are arranged substantially vertically, and the lordosis is formed by the angles of the top portion 111 and bottom portion 112 of external truss structure 105. For example, in the illustrated embodiment, top portion 111 and bottom portion 112 of external truss structure 105 are not perpendicular to the vertical plane defined by the side portion 113. Rather, the top portion 111 and bottom portion 112 are arranged with an acute angle relative to the vertical plane of side portion 113 at or near the anterior region 118 of implant 100 and with an obtuse angle relative to the vertical plane of side portion 113 at or near posterior region 120 of implant 100. As depicted, the vertical struts 103 that form the planar truss of side portion 113 of external truss structure 105 proximate posterior region 120 of implant 100 are shorter than struts 103 that form side portion 113 of external truss structure 105 proximate anterior region 118 of implant 100. In the illustrated embodiment, in which the vertical trusses 103 are substantially evenly spaced, the struts 103 forming the "X" cross members of the side planar trusses proximate the posterior region 120 of implant 100 are shorter than struts forming the "X" cross members of the side planar trusses proximate the anterior region 118 of implant 100. Other embodiments may include variations in the arrangement of the trusses to provide various configurations of the implant. For example, in some embodiments only one or neither of the top and bottom external truss portions may be non-perpendicular to the side portions of the external truss proximate the anterior and posterior portions of the implant. Further, the side, top, and/or bottom portions may include multiple planar trusses angled relative to one another in any orientation. For example, the top or bottom portions may include four planar trusses, each formed of multiple truss units, such that the portion(s) includes a pyramidal like shape.

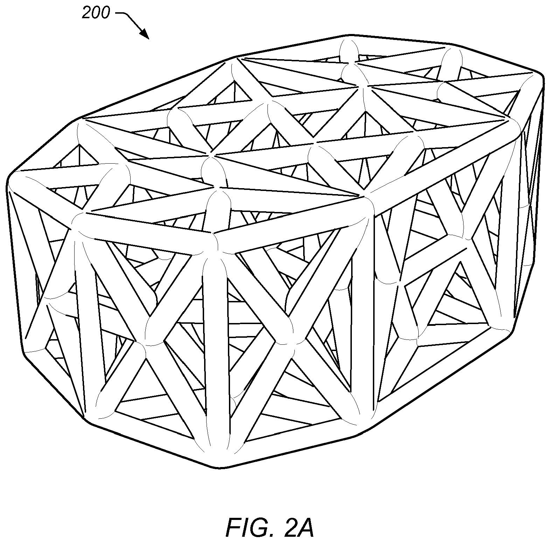

In some embodiments, the implant may not include lordosis. For example, FIGS. 2A-2B illustrate two views of an embodiment of an implant 200 without lordosis. In some embodiments, the top surface and bottom surface may not include connecting struts. For example, FIGS. 2C-2D illustrate two views of implant 250 without outer struts (e.g., without external truss portions formed of planar trusses). In the illustrated embodiment, implant 250 includes internal web structure 104 (e.g., a space truss) and does not include an external truss structure. For example, in the illustrated embodiment, the exterior faces of implant 250 are defined by a plurality of truss units that are angled relative to each of its adjacent truss units. The relative alignment of the truss units results in a non-planar exterior that includes a plurality of pointed junctions. The pointed junctions (e.g., pointed junction 201) may operate to dig into the surrounding bone to hold the implant in place (for example, if the implant is being used in a corpectomy device).

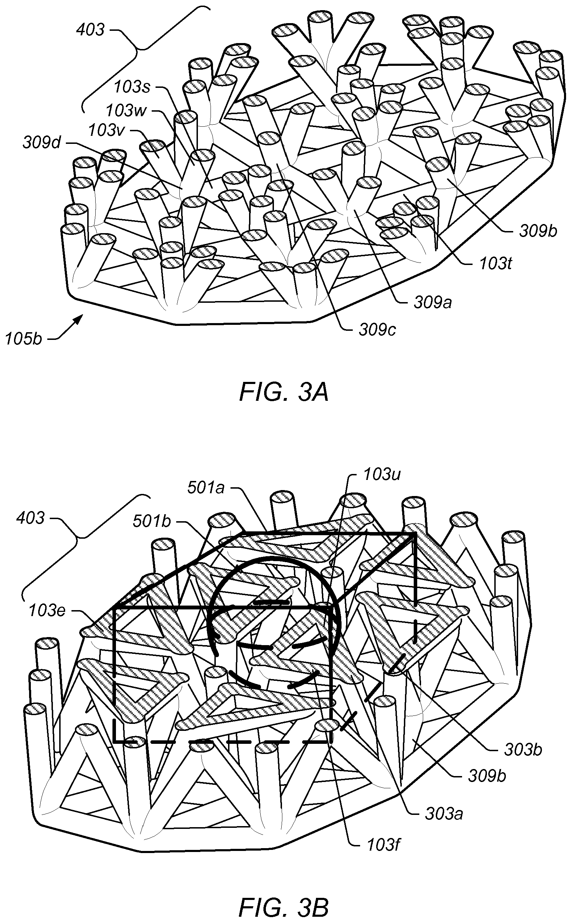

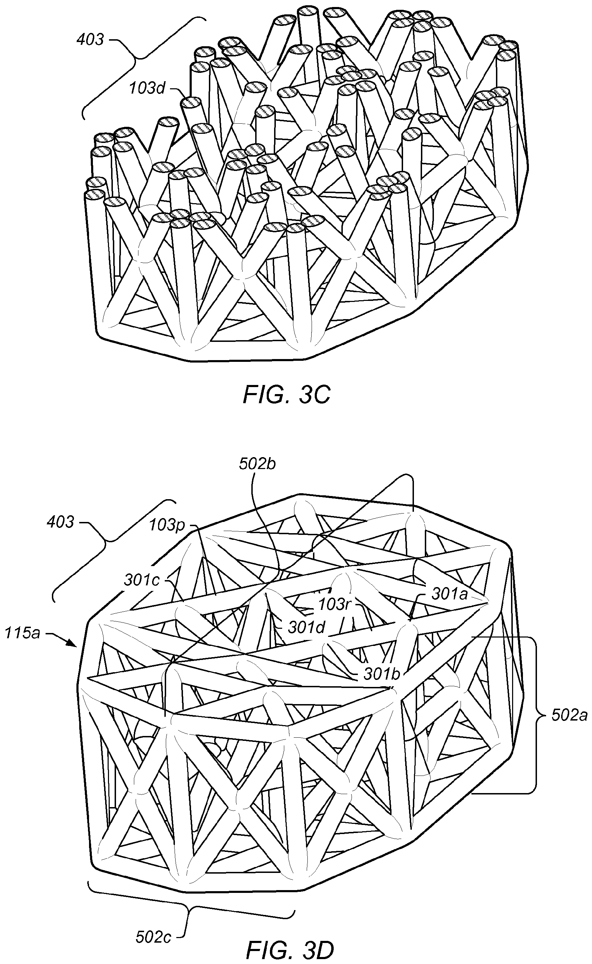

FIGS. 3A-3C illustrate progressive sectioned views of implant 100 showing the internal structure of implant 100, according to an embodiment. FIG. 3A illustrates a sectioned view of a lower portion of implant 100. Bottom surface 105b is shown with various struts (e.g., struts 103) extending upward from bottom surface 105b. FIG. 3B illustrates a sectioned view approximately mid-way through implant 100. Struts, such as struts 103e, 103f, shared by various stacked tetrahedrons in the web structure are shown. Some struts extend through central portion 501a and/or 501b of implant 100. FIG. 3B also shows central portions 501a,b of implant 100. In some embodiments, central portion 501a may include a rectangular region that has a width of approximately 50% of the implant width, a height of approximately 50% of the implant height, and a length of approximately 50% of the implant length and located in the center of implant 100. In some embodiments, central portion 501b may encompass a region (e.g., a spherical region, square region, etc.) of approximately a radius of approximately 1/8 to 1/4 of the width of implant 100 around a position located approximately at one half the width, approximately one half the length, and approximately one-half the height of implant 100 (i.e., the center of implant 100). Other central portions are also contemplated. For example, the central portion may include a square region with a length of one of the sides of the square region approximately ¼ to ½ the width of implant 100 around a position approximately at one half the width, approximately one half the length, and approximately one half the height of the implant. An example height 502a, width 502b, and length 502c, is shown in FIG. 3D. In some embodiments, the height may be up to about 75mm or more. In some embodiments, such as those used for long bone reconstruction, the width and/or length could be approximately 7 inches or longer. In some embodiments, the width, length, and/or height may vary along implant 100 (e.g., the height may vary if the implant includes lordosis). The height may be taken at one of the opposing sides, the middle, and/or may be an average of one or more heights along the length of implant 100. The web structure may extend through central portion 501a,b of the implant (e.g., at least one strut of the web structure may pass at least partially through central portion 501a,b). FIG. 3C illustrates another sectioned view showing sectioned views of top tetrahedrons in the web structure. FIG. 3D shows a complete view of implant 100 including top surface 115a with vertices 301a-d.

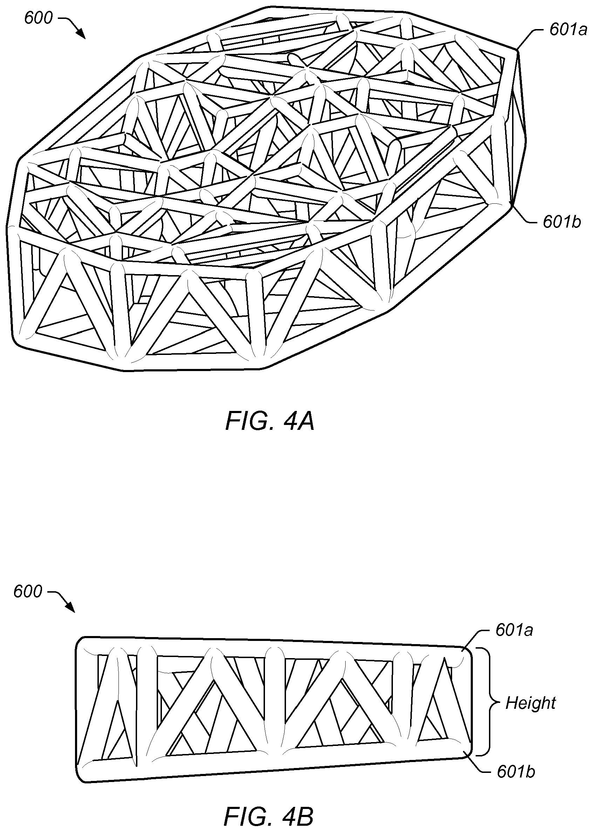

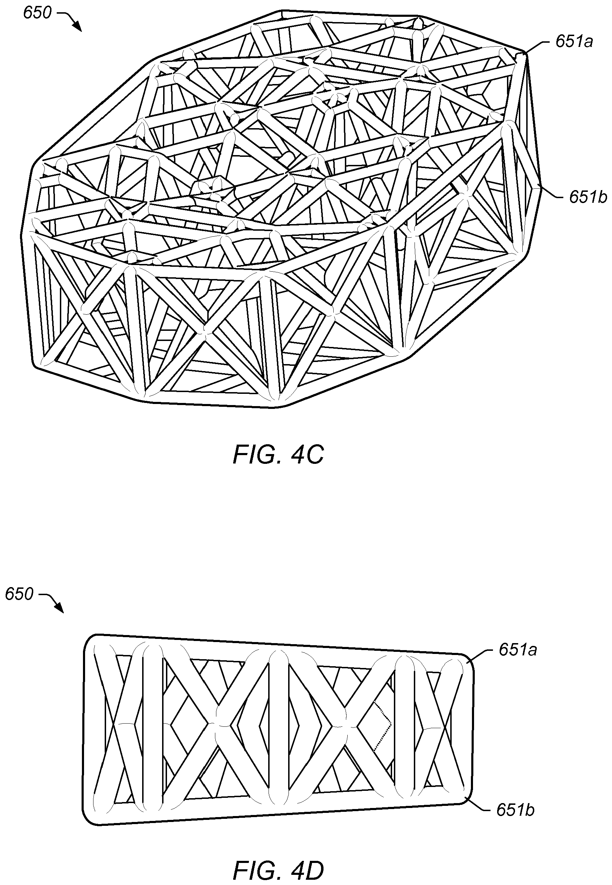

FIGS. 4A-4D illustrate alternate embodiments of implant 100. In some embodiments, different sections of the hexahedron-shaped geometric design may be used. For example, as seen in FIG. 4A, the bottom half of the hexahedron-shaped geometric design may be used (primarily including the lower tetrahedron structures). If using the bottom half of the design, design 600 may be expanded proportionately to have similar overall dimensions as the hexahedron-shaped geometric design (e.g., the tetrahedrons may be expanded to approximately twice the height of the tetrahedrons in the hexahedron-shaped geometric design to give design 600 a height approximately the same as the hexahedron-shaped geometric design). In some embodiments, design 600 may also be angled (e.g., on top surface 601a and/or bottom surface 601b) to provide design 600 with lordosis to, in some embodiments, have a better fit between the vertebral endplates. Top surface 601a and/or bottom surface 601b may also include struts to connect nodes of design 600 (e.g., see the strut network on the top surface in FIG. 4A). Other patterns of struts for top surface 601a and/or bottom surface 601b may also be used. In some embodiments, design 600 may not include negative angles between struts and may thus be easier to create through a casting or molding process.

FIGS. 4C-4D illustrate another alternate embodiment of implant 100. In some embodiments, approximately the middle 40 to 60 percent of the hexahedron-shaped geometric design may be used. For example, if an overall height of the hexahedron-shaped geometric design is approximately 37 mm, approximately the bottom 10 mm and approximately the top 10 mm of the design may be removed and approximately the middle 17 mm of the design may be used for the implant. Middle portion design 650 may then be expanded proportionately such that the approximate height of the expanded design may be approximately 37 mm (or a different height as needed). Top surface 651a and bottom surface 651b may include a network of struts (e.g., see the struts on top surface 651a of FIG. 4C) (other networks of struts are also contemplated). Other portions of the design for the implant are also contemplated (e.g., the top half of the design shown in FIG. 1A, the bottom half of the design shown in FIG. 1A, etc). Design portions may be proportionately expanded to meet specified dimensions (e.g., specified height, width, and length). In some embodiments, the amount of struts may be reduced or material in the implant may be redistributed so that some struts may have a larger diameter and some may have a smaller diameter (e.g., the different diameters may reinforce against different directional forces). In some embodiments, a partial-design cage may be used (e.g., with half of the web structure so that the structure includes a tetrahedron. Further, in some embodiments, the implant may include angled surfaces (e.g., an angled top surface 651a and/or angled bottom surface 651b) to provide lordosis for implants to be implanted between the vertebral endplates.

In some embodiments, the web structure of implant 100 may distribute forces throughout implant 100 when implanted. For example, the connecting struts of the web structure may extend throughout the core of implant 100, and the interconnectivity of struts 103 may disperse the stress of compressive forces throughout implant 100 to reduce the potential of stress risers (the distribution of forces throughout implant 100 may prevent concentration of stress on one or more portions of the vertebrae that may otherwise result in damage to the vertebrae).

In some embodiments, the web structure of implant 100 (e.g., the external and internal struts of implant 100) may also provide surface area for bone graft fusion. For example, the web structure extending throughout implant 100 may add additional surface areas (e.g., on the surface of the struts making up implant 100) to fuse to the bone graft material and prevent bone graft material from loosening or migrating from implant 100. In some embodiments, the web structure may also support bone in-growth. For example, when implanted, adjacent bone (e.g., adjacent vertebrae if the implant is used as a spinal implant) may grow over at least a portion of struts 103 of implant 100. The bone growth and engagement between the bone growth and implant 100 may further stabilize implant 100. In some embodiments, the surfaces of implant 100 may be formed with a rough surface to assist in bone in-growth adhesion.

In some embodiments, struts 103 may have a diameter approximately in a range of about 0.025 to 5 millimeters (mm) (e.g., 1.0 mm, 1.5 mm, 3 mm, etc). Other diameters are also contemplated (e.g., greater than 5 mm). In some embodiments, the struts may have a length approximately in a range of 0.5 to 20 mm (e.g., depending on the implant size needed to, for example, fit a gap between vertebral endplates). As another example, struts may have a length approximately in a range of 30-40 mm for a hip implant. In some embodiments, the reduced strut size of the web structure may allow the open cells in implant 100 to facilitate bone growth (e.g., bone may grow through the open cells once implant 100 is implanted in the body). Average subsidence for implants may be approximately 1.5 mm within the first 3 weeks post op (other subsidence is also possible (e.g., approximately between 0.5 to 2.5 mm)). A strut size that approximately matches the subsidence (e.g., a strut size of approximately 1.5 mm in diameter and a subsidence of approximately 1.5 mm) may result in a net 0 impedance (e.g., the bone growth growing around the struts) after implant 100 has settled in the implanted position. The net 0 impedance throughout the entire surface area of the implant/vertebrae endplate interface may result in a larger fusion column of bone that may result in more stable fusion. Other fusion column sizes are also contemplated. The configuration of the implant 100 may redistribute the metal throughout the implant 100. In some embodiments, a rim may not be included on the implant 100 (in some embodiments, a rim may be included). The resulting bone growth (e.g., spinal column) may grow through the implant 100.

In some embodiments, greater than 50% of the interior volume of implant 100 may be open. In some embodiments, greater than 60%, greater than 70%, and/or greater than 80% of implant 100 may be open (e.g., 95%). In some embodiments, the open volume may be filled with bone growth material. For example, cancellous bone may be packed into an open/internal region of implant 100.

In some embodiments, at least a portion of the surfaces of implant 100 may be coated/treated with a material intend to promote bone growth and/or bone adhesion and/or an anitmicrobial agent to prevent infections. For example, in some embodiments, the surface of the struts (e.g., struts 103 forming the web structure) may be coated with a biologic and/or a bone growth factor. In some embodiments, a biologic may include a coating, such as hydroxyapatite, bone morphaginic protein (BMP), insulinlike growth factors I and II, transforming growth factor-beta, acidic and basic fibroblast growth factor, platelet-derived growth factor, and/or similar bone growth stimulant that facilitates good biological fixation between the bone growth and a surface of the implant. In some embodiments, a bone growth factor may include a naturally occurring substance capable of stimulating cellular growth, proliferation and cellular differentiation (e.g., a protein or steroid hormone).

In some embodiments, a biologic and/or growth factor may be secured to a central region of implant 100. For example, in some embodiments, a biologic or growth factor may be provided on at least a portion of a strut that extends through central portion 501a and/or 501b of implant 100. Such an embodiment may enable the delivery of a biologic and or a growth factor to a central portion of an implant. For example, the biologic or growth factor may be physically secured to a strut in a central portion of implant 100 as opposed to being packed into an open volume that does not include a strut provided therein for the physical attachment of the biologic and/or growth factor.

As implant 100 settles into the implant site, subsidence may place additional pressure on the bone graft material (which may already be under compressive forces in implant 100) and act to push the bone graft material toward the sides of implant 100 (according to Boussinesq's theory of adjacent material, when a force is applied to a member that is adjacent to other materials (such as sand, dirt, or bone graft material) the force against the member creates a zone of increased pressure (e.g., 60 degrees) in the adjacent material). Struts 103 of the web structure may resist bone graft material protrusion from the sides of the web structure and may increase the pressure of the bone graft material. Bone graft material may need to be implanted in a higher-pressure environment to create an environment conducive to strong bone growth (e.g., according to Wolf's law that bone in a healthy person or animal will adapt to the loads it is placed under). The web structure may thus increase the chance of stronger fusion.

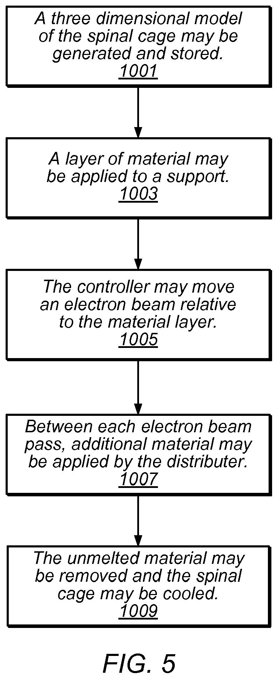

FIG. 5 illustrates a flowchart of a method for making implant 100. In some embodiments, implant 100 may be made through rapid prototyping (e.g., electron beam melting, laser sintering, etc). It should be noted that in various embodiments of the methods described below, one or more of the elements described may be performed concurrently, in a different order than shown, or may be omitted entirely. Other additional elements may also be performed as desired. In some embodiments, a portion or the entire method may be performed automatically by a computer system.

At 1001, a three dimensional model of implant 100 may be generated and stored in a storage medium accessible to a controller operable to control the implant production process. At 1003, a layer of material (e.g., a powder, liquid, etc.) may be applied to a support. In some embodiments, the powder may include γTiAl (γTitanium Aluminides) which may be a high strength/low weight material. Other materials may also be used. The powder may be formed using a gas atomization process and may include granules with diameters approximately in a range of 20 to 200 micrometers (µm) (e.g., approximately 80 µm). The powder may be delivered to the support through a distributer (e.g., delivered from a storage container). The distributer and/or the support may move during distribution to apply a layer (e.g., of powder) to the support. In some embodiments, the layer may be approximately a uniform thickness (e.g., with an average thickness of 20 to 200 micrometers (µm)). In some embodiments, the distributer and support may not move (e.g., the material may be sprayed onto the support). At 1005, the controller may move a directed energy source (e.g., an electron beam, laser, etc.) relative to the material layer. In some embodiments, the directed energy source generator may be moved, and in some embodiments the support may be moved. If the material is γTiAl, a melting temperature approximately in a range of 1200 to 1800 degrees Celsius (e.g., 1500 degrees Celsius) may be obtained between the directed energy source and the material. At 1007, between each directed energy source pass, additional material may be applied by the distributer. At 1009, the unmelted material may be removed and implant 100 may be cooled (e.g., using a cool inert gas). In some embodiments, the edges of the implant may be smoothed to remove rough edges (e.g., using a diamond sander). In some embodiments, the implant may include rough edges to increase friction between the implant and the surrounding bone to increase adhesion of the implant to the bone.

Other methods of making implant 100 are also contemplated. For example, implant 100 may be cast or injection molded. In some embodiments, multiple parts may be cast or injection molded and joined together (e.g., through welding, melting, etc). In some embodiments, individual struts 103 forming implant 100 may be generated separately (e.g., by casting, injection molding, etc.) and welded together to form implant 100. In some embodiments, multiple implants of different sizes may be constructed and delivered in a kit. A medical health professional may choose an implant (e.g., according to a needed size) during the surgery. In some embodiments, multiple implants may be used at the implant site.

In some embodiments, the implant may be customized. For example, three dimensional measurements and/or shape of the implant may be used to construct an implant that distributes the web structure throughout a three-dimensional shape design. As noted in FIG. 5, the three-dimensional shape design of the implant may be entered into a computer system/controller that may control the directed energy source melting process. In some embodiments, the truss design and orientation may be preset or predetermined by the computer system/controller. In some embodiments, a user may select the truss design to use and/or may select the orientation of the trusses in the implant. In some embodiments, the user may enter the outer dimensions of the three dimensional shape and the computer system/controller may generate a three-dimensional design that includes the truss design and orientation. The computer system/controller may generate the three-dimensional design by providing a uniform distribution of the truss design throughout a three-dimensional shape with the outer dimensions provided by the user. In some embodiments, the heights and widths of the trusses used in the design may be proportional to the overall height and width of the three-dimensional shape (e.g., the trusses may have heights approximately equal to ½ the overall height and a width of approximately 1/16 the overall width). Other heights and widths are also contemplated. In some embodiments, the user may provide the height and width and/or the computer system/controller may have default heights and widths to use.

In some embodiments, a truss/web structure may be disposed on at least a portion of an implant to facilitate coupling of the implant to an adjacent structure. For example, where an implant is implanted adjacent a bony structure, one or more truss structures may be disposed on and/or extend from a surface (e.g., an interface plate) of the implant that is intended to contact, and at least partially adhere to, the bony structure during use. In some embodiments, such as those including an intervertebral implant disposed between the end plates of two adjacent vertebrae during, one or more truss structures may be disposed on a contact surface of the intervertebral implant to facilitate bone growth that enhances coupling of the intervertebral implant to the bony structure. For example, a truss structure may include one or more struts that extend from the contact surface to define an open space for bone growth therethrough, thereby enabling bone through growth to interlock the bone structure and the truss structure with one another to couple the implant to the bony structure at or near the contact face. Such interlocking bone through growth may inhibit movement between the implant and the bony structure which could otherwise lead to loosening, migration, subsidence, or dislodging of the implant from the intended position. Similar techniques may be employed with various types of implants, including those intended to interface with tissue and/or bone structures. For example, a truss structure may be employed on a contact surface of knee implants, in a corpectomy device, in a hip replacement, in a knee replacement, in a long bone reconstruction scaffold, or in a cranio-maxifacial implant hip implants, jaw implant, an implant for long bone reconstruction, foot and ankle implants, shoulder implants or other joint replacement implants or the like to enhance adherence of the implant to the adjacent bony structure or tissue.

FIGS. 6A-6C illustrate an embodiment of the invention. In some embodiments, an implant is composed of a web structure 2900 that may have a non-planar upper surface 2910 and/or lower surface 2920. A non-planar surface includes surfaces that have some deviation from planarity. The non-planar upper surface 2910 and lower surface 2920 may extend beyond the height defined by outer trusses 2930 of the implant. In an embodiment, a truss design as described herein may be modified to have a non-planar upper surface 2910 and/or a non-planar lower surface 2920 by extending the length of polar trusses 2950 to expand the central region of the web structure. Forming non-planar upper and/or lower surfaces may help the implant to match better with a non-planar implant site. For example, expanding the central portion of the web structure to create rounded upper and/or lower surfaces may help the implant to match better with a bone surface (or other implant sites) that has a concave feature. As depicted in FIGS. 6A and 6B the non-planar upper surface forms a convex implant surface. It should be understood, that in another embodiment, a non-planar surface may be concave (i.e., the polar trusses of the web structure may be shortened with respect to the height defined by the outer trusses 2930). An implant having a concave exterior profile may be useful for implantation sites having a convex surface. In some embodiments, the web structure may have a convex upper surface and a concave lower surface. Further, in some embodiments, the web structure may include angled surfaces (e.g., an angled top surface 2930a and/or angled bottom surface 2930b) to provide lordosis for implants to be implanted between the vertebral endplates.

In other embodiments, the non-planar surface may have discrete portions of non-planarity. For example, some of the polar trusses may be shortened with respect to the height defined by the outer trusses, while other of the polar trusses may extend beyond the height defined by the outer trusses. This may create a non-planar surface that includes raised and sunken portions. The location of the raised and/or sunken portions of the implant may be selected to match the contour of the implant site.

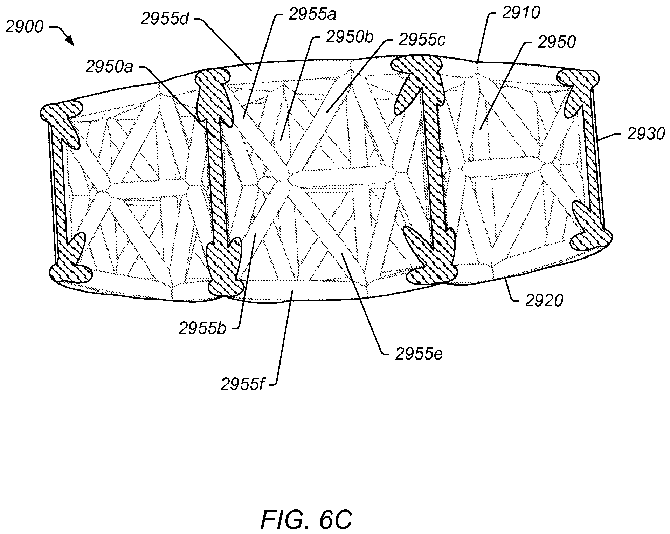

FIG. 6C depicts a cross-sectional view of an implant having a non-polar surface. In an embodiment, the implant includes a web-structure 2900 formed from a plurality of polar struts 2950 that extend from a contact surface of the implant (e.g., 2910) to an opposing contact surface of the implant (e.g., 2920). A plurality of connecting surface struts 2955 are coupled to the ends of polar struts 2950. Together connecting surface struts 2955 and polar struts 2950 define a plurality of planar truss units. In an embodiment, at least two of the polar struts 2950 have different lengths which define a non-planar contact surface for the web structure. For example, polar strut 2950a has a length that is less than the length of polar strut 2950b. By placing the connecting surface struts 2955 to couple the ends of each of the polar struts, the contact surface becomes non-planar. In the depicted embodiment, polar struts 2950b create a central portion of the implant that has a height (thickness) that is greater than the peripheral portion of the implant, defined by polar struts 2950a. The upper and lower contact surface of implant 2900 therefore are non-planar (in this example, rounded) by virtue of extending connecting surface struts 2955 to reach the ends of each of polar struts 2950. In some embodiments, connecting surface struts may be curved to impart a more rounded profile to the contact surface.

In some embodiments, the length of the central polar struts is greater than the length of the peripheral polar struts, creating a convex surface for the implant. In other embodiments, the length of the central polar struts is less than the length of the peripheral polar struts, creating a concave surface for the implant. In other embodiments, the polar struts may have different lengths to create a customized surface profile that is non-planar and may include concave and convex portions.

In some embodiments, the planar truss units are planar triangular truss units. For example, in FIG. 6C, polar strut 2950a and connecting struts 2955a and 2955b together define a planar triangular truss unit. Additional support for the web structure may be obtained using opposing inverted triangular or pyramidal truss units. For example, as shown in FIG. 6C, a first triangular truss unit defined by struts 2955a, 2955c, and 2955d is coupled to a second triangular truss unit defined by struts 2955b, 2955e, and 2955f through node 2960. The first and second triangular truss units are coupled in an opposing manner with the node 2960 defining the apex of each triangular truss unit. It should be understood that while depicted as triangular truss units, pyramidal truss units, composed of four struts (trigonal pyramidal) or five struts (square pyramid), may be used to define a portion of the web structure of an implant.

According to the invention, a non-planar surface of an implant may be defined by a web structure, wherein the web structure includes a plurality of planar truss units coupled to each other, the planar truss units including a plurality of struts coupled to a plurality of nodes. In the invention, one or more angles defined by two struts and a node of one or more planar truss units are different than one or more corresponding angles defined by two struts and a node of one or more other planar truss units, wherein the nodes of the non-equivalent angle planar truss units are coupled to each other with one or more exterior struts, the exterior struts defining a non-planar surface. As used herein the phrase "corresponding angles" refers to the angles formed at similar portions of two planar truss units. For example, if the triangle is a non-equilateral triangle the corresponding angles may be the two base angles of the triangle. The angles of the two adjacent planar truss units are set such that the nodes connecting the struts that define the non-equivalent angles come together at different elevations with respect to other nodes of the web structure to create a non-planar surface as depicted in FIGS. 6A-6C.

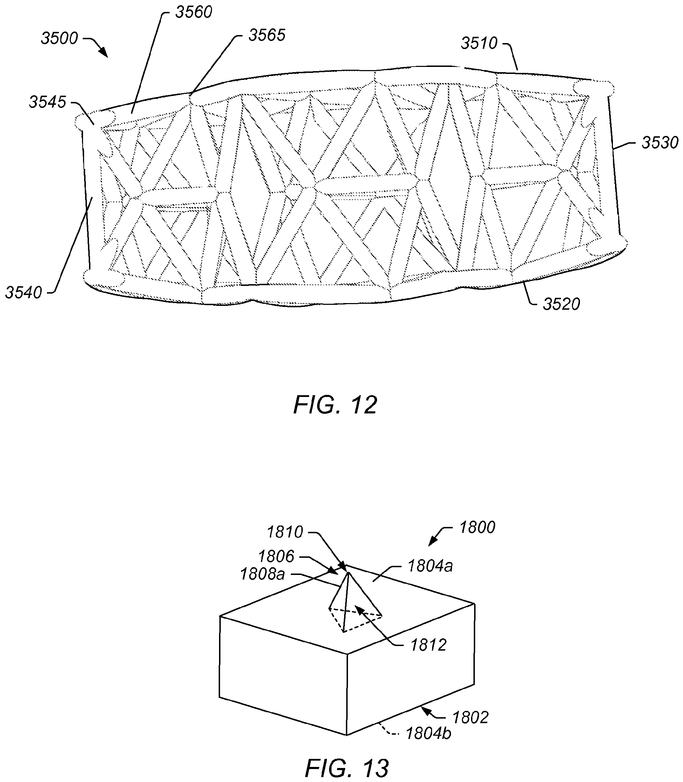

FIG. 12 illustrates another alternate embodiment of an implant. In some embodiments, an implant is composed of a web structure 3500 that may have a non-planar upper contact surface 3510 and/or lower contact surface 3520. A non-planar contact surface includes surfaces that have some deviation from planarity. The non-planar upper contact surface 3510 and lower contact surface 3520 may extend beyond the height defined by outer trusses 3530 of the implant. In an embodiment, a truss design as described herein may be modified to have a non-planar upper contact surface 3510 and/or a non-planar lower contact surface 3520 by altering the angles of struts that extend toward the interior of the implant. In an embodiment, an implant includes a plurality of side surface planar truss units (shown in cross section as 3540) defining an exterior side surface of the implant, the side surface planar truss units are composed of a plurality of struts coupled to a plurality of nodes 3545. A plurality of surface struts 3560 extend from one or more of the nodes 3545 of one or more of the side surface planar truss units toward the interior portion of the implant. To create a non-planar surface, the angle between the surface struts 3560 and the struts of the side surface planar truss units 3540 is made to be substantially greater than 90 to create a raised non-planar surface, or substantially less than 90 to create an indented non-planar surface. A plurality of interior nodes 3565 couple the surface struts to each other to define an exterior contact surface of the implant. The angle of the surface struts is in this manner set such that a non-planar exterior surface is defined by the surface struts and the interior nodes.

In an embodiment, a plurality of planar truss units are coupled to one another such that one or more planar truss units lie in a plane that is not substantially parallel to a plane of a planar truss unit that shares at least one strut with the one or more planar truss units. Some of the planar truss units may be arranged to define an exterior surface of the web structure 2900. The exterior surface of the web structure may be defined by a plurality of planar truss units arranged substantially parallel to a longitudinal axis of the polar struts.

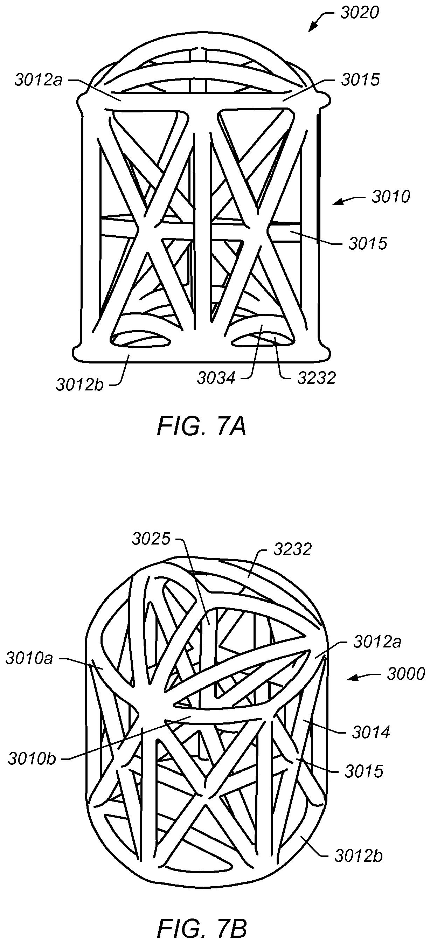

In another embodiment, an implant 3000 is composed of an internal web structure as depicted in FIGS. 7A and 7B. In an embodiment, implant 3000 includes an external truss structure 3010 composed of a plurality of planar trusses, constructed with curved/arch truss members 3012 and straight members 3014 that are coupled about an exterior portion of the implant. For example, in the illustrated embodiment, the external truss structure 3010 includes a series of planar trusses 3010a, 3010b that are coupled to one another. Each planar truss 3010a,b includes a plurality of straight truss units 3014 that are coupled to one another and lie substantially in the same plane. Curved/arch truss members 3012 are coupled to vertices of straight truss units 3014. As depicted, a portion of the straight truss units 3014 includes four triangular planar truss units having a common vertex 3015 and arranged to form a generally rectangular structure that lies in a single common plane. In other words, some of the straight truss units are arranged to form a substantially rectangular structure having "X" shaped struts extending from one corner of the rectangular structure to the opposite corner of the rectangular structure. The curved/arched truss members 3012 are joined together at the vertices of the rectangular structures to form a generally rounded implant member 3000.

Implant member 3000 also includes, in some embodiments, a rounded upper surface and/or a rounded lower surface. Rounded upper surface 3020 may be a convex upper contact surface, as depicted in FIGS. 7A and 7B. It should be understood, however that both the upper and lower surfaces may be convex, or both the upper and lower surfaces may be concave. A convex surface, may be formed by extending central truss 3025, above the height of the implant defined by the planar trusses 3010, and specifically above the height defined by the upper curved/arched truss members 3012a. One or more curved arched truss members 3022 may extend from one or more vertices of the planar trusses 3010, and are coupled together at the extended central truss 3025. The rounded upper surface may be further defined by spanning trusses 3024 that span alternating vertices of the planar trusses 3010.

In a similar manner, a concave rounded lower contact surface may be defined. A concave surface, may be formed by reducing central truss 3025, such that central truss does not extend to the rim defined by the lower curved/arched truss members 3012b. One or more curved arched truss members 3032 may extend from one or more vertices of the planar trusses 3010, and are coupled together at the central truss 3025. The concave lower contact surface may be further defined by spanning trusses 3034 that span alternating vertices of the planar trusses 3010.

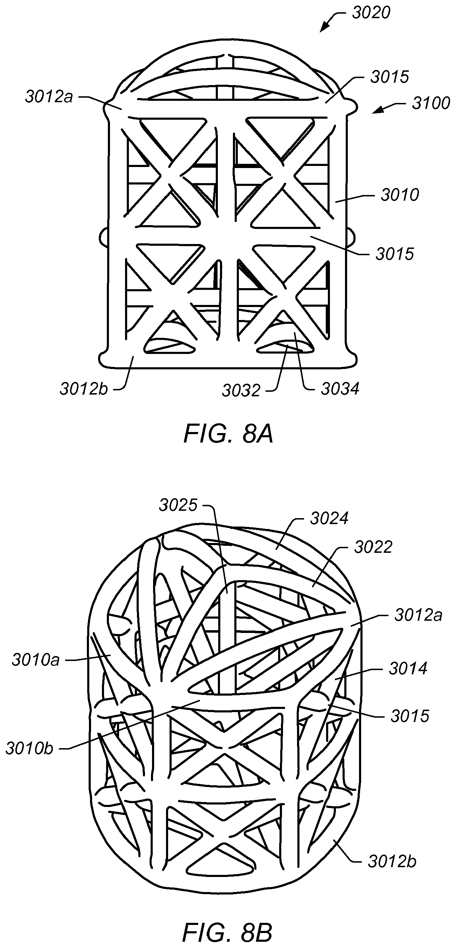

FIGS. 8A and 8B depicted an alternate embodiment of an implant 3100 that includes an internal web structure that is at least partially enclosed by an external truss structure. In an embodiment, external truss structure 3010 includes a plurality of planar trusses, constructed with curved/arched truss members 3012 and straight members 3014 that are coupled about an exterior portion of the implant, as described in FIGS. 7A and 7B. In the embodiments described in FIGS. 8A and 8B, a pair of planar truss forms each segment of the exterior, rather than a single planar truss, as described in FIGS 7A and 7B. Implant 3100 also includes a convex upper surface 3020 and a concave lower surface.

When fusing joints many surgical processes lead to shortening of the anatomy at the region being fused. This is generally seen in both primary and revision fusions. Bone revision fusions are performed to correct portions of bone not healed at all (non-union) or bone healed in the wrong position (mal-union). Problems that arise from bone primary and revision fusions include alteration of biomechanics of the effected area. The alterations lead to the patient's biomechanics being changed to compensate for the alterations. Additionally, cosmetic changes may be visible that lead to patient dissatisfaction. The current standard is to either accept the shortening and its consequences or attempt to preserve length with bone block grafting (allograft or autograft) or bone transport. Disadvantages to bone block grafting include added morbidity associated with taking autograft from the pelvis and difficulty of the body incorporating the graft into the fusion site. Allograft bone blocks are also difficult to incorporate into the region. Typically procedures for correcting length include boney transport that requires external fixation and extended periods of non-weight bearing while length is obtained. Pin tract infections and unsightly sinus tracts may also a problem with boney transport.

In an embodiment, one or more implants, as described herein, may be used to fill a void between bone portions in a way that reduces morbidity, reduces healing time, and improves the fusion between the bone portions. In one embodiment, a method of coupling bone portions includes placing an implant, for example, as described in FIGS. 7A, 7B, 8A, or 8B, in contact with the exposed ends of the bone. The bone fusion site is exposed to allow insertion of the implant. Joint preparation for the fusion is used to preserve length, adjust alignment and provide a stable construct. In an embodiment, one or both portions of the joint are reamed to provide a surface that is congruent with the surfaces of the implant. In order to provide stability to implant 3000, the implant is placed on the bone on the complementary surfaces, e.g., the concave surface of the implant is placed on a convex surface of the bone. Autograf, along with stem cells, may be packed in the implant. A traditional fusion plate is also used at the fusion site to maintain the bone position as the fusion heals. Additional autograft, along with stem cells, may be packed around the implant. After the bone is fused, the fusion plates may be removed. It has been shown that this procedure may successfully restore and/or maintain the length of the bone.

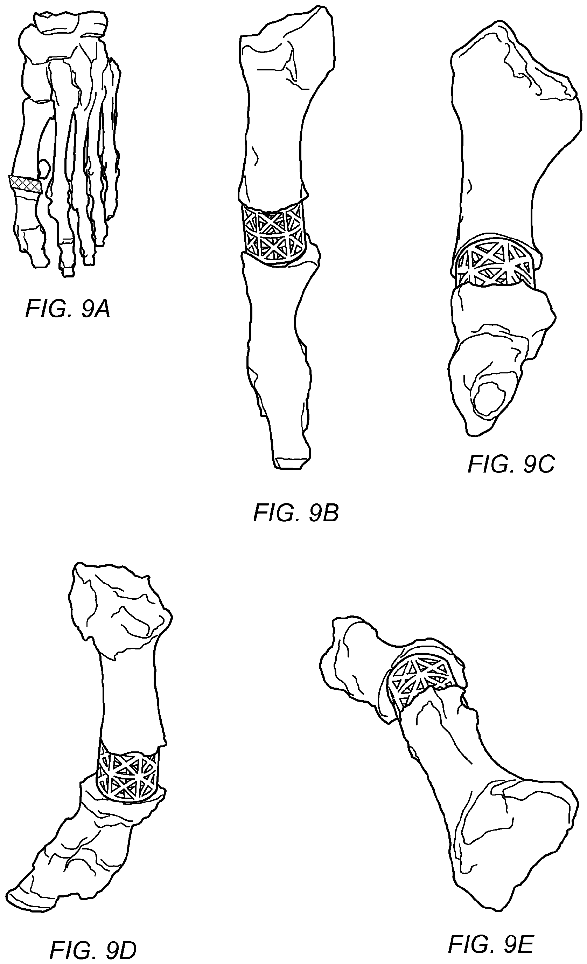

The bone fusion method described above may be used for a number of bone fusion applications. Some examples of sites that may be used for bone fusions are depicted in FIGS. 9A-E. FIG. 9A depicts an implant (3000) used in a metatarsal phalangeal joint replacement. In an embodiment of a metatarsal phalangeal joint replacement process, bone marrow aspirate is obtained from the proximal tibia along with cancellous autograft thru a small incision and small boney window. The bone marrow is then prepared in a centrifuge where the stem cells are concentrated for later reinjection into the fusion site, which enhances the fusion. The cancellous autograft is then tightly packed into the implant (e.g., implant 3000) and then soaked in the stem cells. A dorsal approach is made to the first metatarsal phalangeal joint in standard fashion. Joint preparation for the fusion is used to preserve length, adjust alignment and provide a stable construct. The metatarsal side of the joint (proximal) is reamed to a convex surface. Traditionally the collar of bone around to convexly reamed metatarsal side of the joint is removed to provide a conical congruent surface. In order to provide stability to the implant, the collar of bone is maintained and the concave end of the cage is impacted onto the convex boney surface with the surrounding boney collar intact, which enhances stability and boney incorporation. The phalangeal side of the joint (distal) is reamed in a concave fashion in order to accommodate the convex end of the implant. Traditionally the phalangeal side is symmetrically reamed. With the implant, a groove is reamed in a trough like fashion on the concavely reamed surface to enhance stability and aid in adjusting for plantar and dorsal flexion of the fusion site. A coupling member (e.g., a traditional fusion plate) is placed and additional autograft along with stem cells are packed around the implant. The wound is closed in standard fashion.

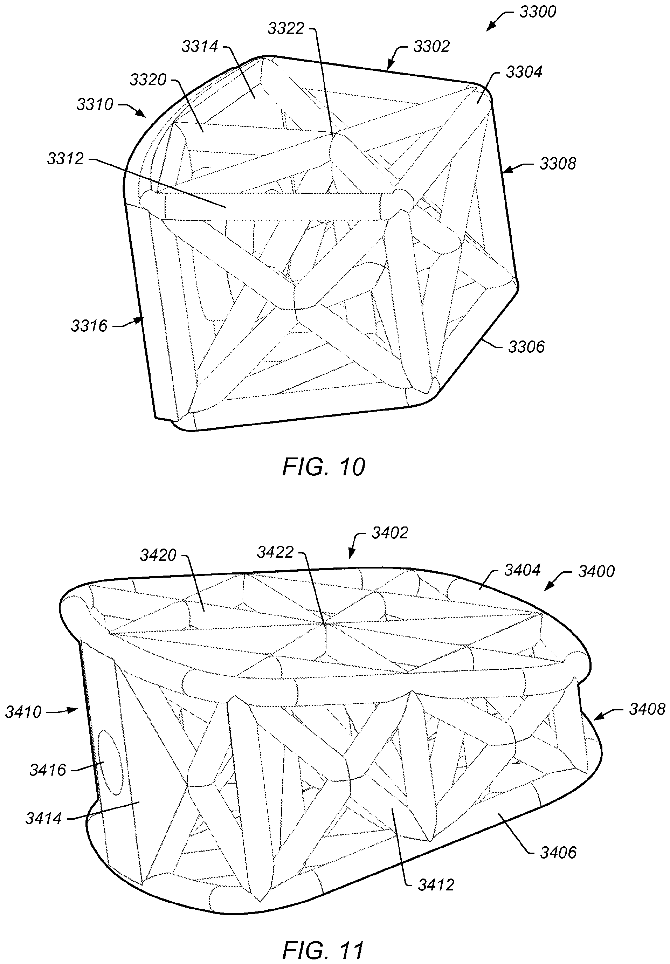

FIG. 10 illustrates a perspective view of an alternate design of an implant. Implant 3300 may include a wedge-shaped body 3302. In some embodiments, body 3302 may include a web/truss structure similar to that described above. In some embodiments, body 3302 may include an upper face 3304 and a lower face 3306. In some embodiments, upper face 3304 and lower face 3306 may be angled relative to one another. In some embodiments, upper face 3304 and/or lower face 3306 may include a substantially flat/planar shape formed of a plurality of struts. In some embodiments, implant 3300 may include a first end 3308 that is tapered. For example, first end 3308 may have a width that is less than a second end 3310, opposite first end 3308. In some embodiments, first end 3308 and/or second end 3310 may include a rounded/curved shape. In some embodiments, first end 3308 and/or second end 3310 may include a substantially flat/planar shape formed of a plurality of struts. In some embodiments, sides 3312 may include a substantially flat/planar shape formed of a plurality of struts. In some embodiments, first end 3308 and/or second end 3310 may include one or more tool engagement features 3316. Tool engagement feature 3316 may be coupled to or otherwise engaged by a tool used for placement of implant 3300. In some embodiments, first end 3308 and/or second end 3310 may include a planar surface structure 3314 spanning an area between struts. In comparison to implant 2700, implant 3300 include additional supporting struts 3320 coupled to the central node 3322. Supporting strut 3320 may provide additional strength to the implant 3300.

FIG. 11 illustrates a perspective view of an alternative embodiment of Evans wedge foot/ankle implant 3400 in accordance with one or more embodiments of the present technique. Implant 3400 is similar in design to implant 2600, having additional support struts formed on the top and bottom surfaces. Implant 3400 may include a wedge-shaped body 3402. In some embodiments, body 3402 may include a web/truss structure similar to that described above. In some embodiments, body 3402 may include an upper face 3404 and a lower face 3406. In some embodiments, upper face 3404 and lower face 3406 may be angled relative to one another. In some embodiments, upper face 3404 and/or lower face 3406 may include a substantially flat/planar shape formed of a plurality of struts. In some embodiments, implant 3400 may include a first/front end 3408 that is tapered. For example, first end 3408 may have a width that is less than a second/rear end 3410, opposite first end 3408. In some embodiments, first end 3408 and/or second end 3410 may include a rounded/curved shape. In some embodiments, sides 3412 may include a substantially flat/planar shape formed of a plurality of struts. In some embodiments, first end 3408 and/or second end 3410 may include a planar surface structure 3414 spanning an area between struts.

In some embodiments, first end 3408 and/or second end 3410 may include one or more tool engagement features 3416. Tool engagement feature 3416 may be coupled to or otherwise engaged by a tool used for placement of implant 3400. In comparison to implant 2600, implant 3400 include additional supporting struts 3420 coupled to the node 3422. Supporting struts 3420 may provide additional strength to the implant 3400.

FIG. 13 depicts an embodiment of an implant 1800 in accordance with one or more embodiments of the present technique. In some embodiments, implant 1802 may include a spinal implant, a knee implant, a hip implant, a jaw implant, an implant for long bone reconstruction, ankle implant or the like. In the illustrated embodiment, implant 1800 includes a body 1802 having two contact faces 1804a,b. As used herein, the term "contact face" refers to a portion of an implant intended to be in contact or near contact with an adjacent structure (e.g., a bony structure) to adhere/couple with the adjacent structure when implanted. A contact surface may include an interface plate of an implant, for instance. An implant may include any number of contact faces. For example an implant may include one or more contact faces intended to couple to one or more adjacent bony structures. As depicted, in some embodiments, contact face 1804a may include an upper contact face intended to contact and secure to a first adjacent bony structure, and 1804b may include a lower contact face intended to contact and secure to a second adjacent bony structure. For example, where implant 1800 is intended to sandwich between two adjacent bony structures (e.g., end plates of two adjacent vertebrae), contact face 1804a may couple to a portion of the first bony structure disposed above implant 1800 and contact face 1804b may couple to the second bony structure disposed below implant 1800. It will be appreciated that the number and orientation of the contact surfaces may vary based on the intended application, and, thus, relative terms such as upper and lower are intended as exemplary and are not intended to be limiting. For example, one or both of the upper and lower contact faces 1804a,b may be oriented such that the are disposed laterally (e.g., as right, left, back and/or front sides of implant body 1802. Moreover, the cubic shape of body 1802 is intended to be exemplary and is not intended to be limiting. For example, body 1802 may include any desirable implant construct such as fusion cages with different shapes or a mechanical construct that allows for motion preservation. Contact surface(s) may take any suitable shape, e.g., a substantially flat planar surface, a curved/contoured surface, ridges, or the like.

In some embodiments, a single, a plurality or all of the contact faces of an implant may include one or more truss structures. In some embodiments, a truss structure includes one or more struts that extend from a respective contact surface and defines an opening that enables bone through growth to facilitate coupling of the truss structure and the implant to the boney structure. For example, in the illustrated embodiment, truss structure 1806 includes a space truss formed of three struts 1807a,b,c that each include elongate members each having a first end coupled to contact surface 1804a and a second end coupled to each of the other struts at a vertex 1810. Each face of the triangular shaped truss structure includes a planar truss structure having a triangular opening with a perimeter defined by two of struts 1807a,b,c and the adjacent portion of contact face 1804a. As depicted, truss structure 1806 includes a generally triangular shaped space truss that defines a four sided, substantially open volume 1812.

In some embodiments, open volume 1812 may facilitate bone growth through truss structure 1806, thereby enhancing coupling of implant 1800 to the adjacent bony structure. For example, in some embodiments, at least a portion of truss structure 1806 is in contact or near contact with the adjacent bony structure, thereby enabling bone growth to extend into and/or through at least a portion of open volume 1812 of truss structure 1806 such that the bone growth interlocks with one or more struts 1808a,b,c of truss structure 1806. The interlocking of the bone growth and the struts may rigidly fix implant 1800 in a fixed location relative to the boney structure.

In some embodiments, implant 1800 may be pressed into contact with the adjacent bony structure such that at least a portion of truss structure 1806 is disposed inside of the adjacent bony structure upon implantation. For example, in some embodiments, implant 1800 may be pressed into contact with the adjacent bony structure such that vertex 1810 pierces into the bony structure and is advanced such that at least a portion of struts 1808a,b,c and open volume 1812 extend into the bony structure. Such a technique may encourage bone to grow into and/or through open volume 1812. In some embodiments, implant 1800 may be advanced/pressed into the adjacent bony structure until the respective contact surface (e.g., upper contact surface 1804a) is in contact or near contact with the adjacent bony structure. In some embodiments, at least a portion of the truss structure and/or the contact surface may be coated/treated with a material intend to promote bone growth and/or bone adherence and an antimicrobial to prevent infection to the truss structure and/or the contact surface. For example, in some embodiments, the surface of the struts and/or the contact surface may be coated with a biologic and/or a bone growth factor, such as those described herein.

In some embodiments, at least a portion of the adjacent bony structure in which the truss structure is to be implanted may be pierced/cut/slit prior to truss structure 1806 being advanced/pressed into the adjacent bony structure. In some embodiments, a cutting tool/edge may be used to cut into the adjacent bony structure such that the resulting cuts accommodate one or more struts of truss structure 1806. For example, where truss structure 1806 includes a triangular shape, such as that depicted in FIG. 13, one or more complementary cuts may be made into the adjacent bony structure in a complementary pattern.

In accordance with the above descriptions, in various embodiments, an implant may include a web structure. The web structure for the implant may include a micro truss design. In some embodiments, the micro truss design may include a web structure with multiple struts. Other web structures are also contemplated. The web structure may extend throughout the implant (including a central portion of the implant). The web structure may thus reinforce the implant along multiple planes (including internal implant load bearing) and provide increased area for bone graft fusion. The web structure may be used in implants such as spinal implants, corpectomy devices, hip replacements, knee replacements, long bone reconstruction scaffolding, hand and wrist bone repair, and cranio-maxifacial implants. Other implant uses are also contemplated. In some embodiments, the web structure for the implant may include one or more geometric objects (e.g., polyhedrons). In some embodiments, the web structure may not include a pattern of geometrical building blocks (e.g., an irregular pattern of struts may be used in the implant). In some embodiments, the web structure may include a triangulated web structure including two or more tetrahedrons. A tetrahedron may include four triangular faces in which three of the four triangles meet at each vertex. The web structure may further include two tetrahedrons placed together at two adjacent faces to form a web structure with a hexahedron-shaped frame (including six faces). In some embodiments, multiple hexahedron-shaped web structures may be arranged in a side-by-side manner. The web structures may connect directly through side vertices (e.g., two or more hexahedron-shaped web structures may share a vertex). In some embodiments, the web structure may be angled to provide lordosis to the implant.

Further modifications and alternative embodiments of various aspects of the invention may be apparent to those skilled in the art in view of this description. For example, although in certain embodiments, struts have been described and depicts as substantially straight elongated members, struts may also include elongated members curved/arched along at least a portion of their length. Accordingly, this description is to be construed as illustrative only and is for the purpose of teaching those skilled in the art the general manner of carrying out the invention. It is to be understood that the forms of the invention shown and described herein are to be taken as embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims. Furthermore, it is noted that the word "may" is used throughout this application in a permissive sense (i.e., having the potential to, being able to), not a mandatory sense (i.e., must). The term "include", and derivations thereof, mean "including, but not limited to". As used in this specification and the claims, the singular forms "a", "an" and "the" include plural referents unless the content clearly indicates otherwise. Thus, for example, reference to "a strut" includes a combination of two or more struts. The term "coupled" means "directly or indirectly connected".

## Claims

1. An implant comprising a web structure (2900), wherein the web structure comprises:
a plurality of planar truss units coupled to each other, the planar truss units comprising a plurality of struts coupled to a plurality of nodes;
**characterised in that** web structure further comprises:
a plurality of internal polar struts (2950) extending from one contact surface (2910) of the implant to an opposing contact surface (2920) of the implant; and wherein exterior connecting struts (2955) couple the ends of the internal polar struts, and wherein at least two of the internal polar struts have different lengths such that the web structure has at least one non-planar surface defined by the ends of the internal polar struts and the exterior connecting struts;
wherein a plurality of planar truss units (2930) define an exterior surface of the web structure, and wherein the exterior surface of the web structure defined by the plurality of planar truss units is substantially parallel to the longitudinal axis of the internal polar struts; and
wherein one or more angles defined by two struts and a node of one or more planar truss units are different than one or more corresponding angles defined by two struts and a node of one or more other planar truss units, wherein the exterior connecting struts couple the nodes of the non-equivalent angle planar truss units to each other, defining said non-planar contact surface.

2. The implant of claim 1, wherein the plurality of planar truss units comprise one or more planar triangular truss units having three substantially straight struts and three nodes in a triangular configuration.

3. The implant of claim 1 or 2, wherein the plurality of planar truss units are coupled to one another such that one or more planar truss units lie in a plane that is not substantially parallel to a plane of a planar truss unit that shares at least one strut with the one or more planar truss units.

4. The implant of any one of claims 1-3, wherein a plurality of planar truss units define an exterior surface of the web structure.

5. The implant of any one of claims 1-4, wherein the at least some of the exterior connecting struts (2955) define triangular trusses having at least one node shared by two different triangular planar truss units having different corresponding angles.

6. The implant of any one of claims 1-5, wherein the plurality of planar truss units comprises a first planar triangular truss unit (2955a, 2955c, 2955d) coupled to a second planar triangular truss unit (2955b, 2955e, 2955f), wherein the first and second planar triangular truss units are coupled in an opposing manner with a single node (2960) defining the apex of each planar triangular truss unit.

7. The implant of any one claims 1-6, wherein the non-planar surface is a substantially rounded surface.

8. The implant of any one of claims 1-7, wherein opposing surfaces of the implant are rounded.

9. The implant of any one of claims 1-8, wherein one or more of the connecting exterior surface struts are curved to form a rounded exterior surface of the implant.

10. The implant of any one of claims 1-9, wherein at least a portion of the one or more of the planar truss units extend beyond the non-planar surface.

11. The implant of claim 1, wherein the at least some of the exterior connecting struts (2955) define triangular trusses having at least one node at the end of a polar strut.

12. The implant of claim 1 or 11, wherein at least a portion of the struts coupled to a node at the end of a polar strut are curved to form a rounded exterior surface of the implant.

13. The implant of any one of claims 1-10, wherein the plurality of planar truss units define a plurality of side surface planar truss units defining an exterior side surface of the implant, the side surface planar truss units comprising a plurality of struts coupled to a plurality of nodes; and wherein a plurality of surface struts extend from one or more of the nodes of one or more of the side surface planar truss units toward the interior portion of the implant, wherein the angle between the surface struts and the struts defining the side surface planar truss units is substantially greater than, or substantially less than 90 ; and wherein the implant further comprises a plurality of interior nodes coupling the surface struts to each other to define an exterior surface of the implant.

## Patentansprüche

1. Implantat mit einer Gewebestruktur (2900), wobei die Gewebestruktur umfasst:
mehrere plane Trägereinheiten, die miteinander gekoppelt sind, wobei die planen Trägereinheiten mehrere Streben umfassen, die mit mehreren Knoten verbunden sind;
**dadurch gekennzeichnet, dass** die Gewebestruktur ferner umfasst:
mehrere interne polare Streben (2950), die von einer Kontaktfläche (2910) des Implantats zu einer gegenüberliegenden Kontaktfläche (2920) des Implantats verlaufen; und wobei äußere Verbindungsstreben (2955) die Enden der internen polaren Streben verbinden, und wobei mindestens zwei der internen polaren Streben unterschiedliche Längen aufweisen, derart, dass die Gewebestruktur mindestens eine nicht-plane Fläche aufweist, die durch die Enden der internen polaren Streben und die äußeren Verbindungsstreben definiert ist;
wobei mehrere plane Trägereinheiten (2930) eine äußere Fläche der Gewebestruktur definieren und wobei die äußere Fläche der Gewebestruktur, die durch die mehreren planen Trägereinheiten definiert ist, im Wesentlichen parallel zur Längsachse der internen polaren Streben ist; und
wobei einer oder mehrere Winkel, die durch zwei Streben und einen Knoten einer oder mehrerer planer Trägereinheiten definiert sind, anders sind als einer oder mehrere entsprechende Winkel, die durch zwei Streben und einen Knoten einer oder mehrerer anderer planer Trägereinheiten definiert sind, wobei die äußeren Verbindungsstreben die Knoten der planen Trägereinheiten mit nicht-äquivalentem Winkel miteinander verbinden, wodurch die nicht-plane Kontaktfläche definiert wird.

2. Implantat nach Anspruch 1, wobei die mehreren planen Trägereinheiten eine oder mehrere plane dreieckige Trägereinheiten umfassen, die drei im Wesentlichen gerade Streben und drei Knoten in einer dreieckigen Konfiguration aufweisen.

3. Implantat nach Anspruch 1 oder 2, wobei die mehreren planen Trägereinheiten derart miteinander verbunden sind, dass eine oder mehrere plane Trägereinheiten in einer Ebene liegen, die nicht im Wesentlichen parallel zu einer Ebene einer planen Trägereinheit ist, die mindestens eine Strebe mit der einen oder den mehreren planen Trägereinheiten teilt.

4. Implantat nach einem der Ansprüche 1 bis 3, wobei mehrere plane Trägereinheiten eine äußere Fläche der Gewebestruktur definieren.

5. Implantat nach einem der Ansprüche 1 bis 4, wobei die mindestens einigen der äußeren Verbindungsstreben (2955) dreieckige Träger mit mindestens einem Knoten definieren, der von zwei verschiedenen dreieckigen planen Trägereinheiten mit unterschiedlichen entsprechenden Winkeln geteilt wird.

6. Implantat nach einem der Ansprüche 1 bis 5, wobei die mehreren planen Trägereinheiten eine erste plane dreieckige Trägereinheit (2955a, 2955c, 2955d) umfassen, die mit einer zweiten planen dreieckigen Trägereinheit (2955b, 2955e, 2955f) gekoppelt ist, wobei die erste und die zweite plane dreieckige Trägereinheit einander gegenüberliegend mit einem einzelnen Knoten (2960) verbunden sind, der den Scheitel jeder planen dreieckigen Trägereinheit definiert.

7. Implantat nach einem der Ansprüche 1 bis 6, wobei die nicht-plane Fläche eine im Wesentlichen abgerundete Fläche ist.

8. Implantat nach einem der Ansprüche 1 bis 7, wobei gegenüberliegende Flächen des Implantats abgerundet sind.

9. Implantat nach einem der Ansprüche 1 bis 8, wobei eine oder mehrere der Verbindungsstreben an der äußeren Fläche gekrümmt sind, um eine abgerundete äußere Fläche des Implantats zu bilden.

10. Implantat nach einem der Ansprüche 1 bis 9, wobei mindestens ein Abschnitt der einen oder mehreren der planen Trägereinheiten über die nicht-plane Fläche hinausragt.

11. Implantat nach Anspruch 1, wobei die mindestens einigen der äußeren Verbindungsstreben (2955) dreieckige Träger mit mindestens einem Knoten am Ende einer polaren Strebe definieren.

12. Implantat nach Anspruch 1 oder 11, wobei mindestens ein Abschnitt der Streben, die mit einem Knoten am Ende einer polaren Strebe verbunden sind, gekrümmt ist, um eine abgerundete äußere Fläche des Implantats zu bilden.

13. Implantat nach einem der Ansprüche 1 bis 10, wobei die mehreren planen Trägereinheiten mehrere plane Seitenflächen-Trägereinheiten definieren, die eine äußere Seitenfläche des Implantats definieren, wobei die planen Seitenflächen-Trägereinheiten mehrere Streben umfassen, die mit mehreren Knoten verbunden sind; und wobei mehrere Oberflächenstreben von einem oder mehreren der Knoten einer oder mehrerer der planen Seitenflächen-Trägereinheiten zum inneren Abschnitt des Implantats verlaufen, wobei der Winkel zwischen den Oberflächenstreben und den Streben, welche die planen Seitenflächen-Trägereinheiten definieren, wesentlich größer als oder wesentlich weniger als 90 ist; und wobei das Implantat ferner mehrere innere Knoten umfasst, welche die Oberflächenstreben miteinander verbinden, um eine äußere Fläche des Implantats zu definieren.

## Revendications

1. Implant comprenant une structure en toile (2900), dans lequel la structure en toile comprend :
une pluralité d'unités planes de type treillis couplées les unes aux autres, les unités planes de titrer comprenant une pluralité d'entretoises couplées à une pluralité de noeuds ;
**caractérisé en ce que** la structure du toit comprend en outre :
une pluralité d'entretoises polaires internes (2950) s'étendant à partir d'une surface de contact (2910) de l'implant en direction d'une surface de contact opposée (2920) de l'implant ; et dans lequel des entretoises de connexion externe (2955) se couplent aux extrémités des entretoises polaires internes, dans lequel au moins deux des entretoises polaires internes possèdent des longueurs différentes de sorte que la structure en toile possède au moins une surface non plane définie par les extrémités des entretoises polaires internes et par les entretoises de connexion externes ;
dans lequel une pluralité d'unités planes de type treillis (2930) définissent une surface externe de la structure en toile, et dans lequel la surface externe de la structure en toile définie par la pluralité d'unités planes de type treillis est essentiellement parallèle à l'axe longitudinal des entretoises polaires internes ; et
dans lequel un ou plusieurs angles définis par deux entretoises et un noeud d'une ou de plusieurs unités planes de type treillis sont différents d'un ou de plusieurs angles correspondants définis par deux entretoises et un noeud d'une ou de plusieurs autres unités planes de type treillis, dans lequel les entretoises de connexion externes se couplent les unes avec les autres aux noeuds des unités planes de type treillis d'angle non équivalent, définissant ladite surface de contact non plane.

2. Implant selon la revendication 1, dans lequel la pluralité d'unités planes de type treillis comprend une ou plusieurs unités triangulaires planes de type treillis possédant trois entretoises essentiellement linéaires et trois noeuds dans une configuration triangulaire.

3. Implant selon la revendication 1 ou la revendication 2, dans lequel la pluralité d'unités planes de type treillis sont couplées l'une à l'autre de sorte qu'une ou plusieurs unités planes de type treillis se trouvent dans un plan qui n'est essentiellement pas parallèle à un plan d'une unité plane de type treillis qui partage au moins une entretoise avec l'une ou plusieurs unités planes de type treillis.

4. Implant selon l'une quelconque des revendications 1 à 3, dans lequel une pluralité d'unités planes de type treillis définit une surface externe de la structure en toile.

5. Implant selon l'une quelconque des revendications 1 à 4, dans lequel l'au moins quelques des entretoises de connexion externe (2955) définissent des treillis triangulaires possédant au moins un noeud partagé par deux unités planes triangulaires différentes de type treillis possédant des angles correspondants différents.

6. Implant selon l'une quelconque des revendications 1 à 5, dans lequel la pluralité d'unités planes de type treillis comprend une première unité triangulaire plane de type treillis (2955a, 2955c, 2955d) couplée à une seconde unité triangulaire plane de type treillis (2955b, 2955e, 2955f), dans lequel les premières et seconde unité triangulaire plane de type treillis sont couplées de façon opposée avec un noeud unique (2960) définissant le sommet de chaque unité triangulaire plane de type treillis.

7. Implant selon l'une quelconque des revendications 1 à 6, dans lequel la surface non plane consiste en une surface essentiellement arrondie.

8. Implant selon l'une quelconque des revendications 1 à 7, dans lequel des surfaces opposées de l'implant sont arrondies.

9. Implant selon l'une quelconque des revendications 1 à 8, dans lequel une ou plusieurs des entretoises de surface extérieure de connexion sont courbes pour former une surface extérieure arrondie de l'implant.

10. Implant selon l'une quelconque des revendications 1 à 9, dans lequel au moins une partie de l'une ou plusieurs des unités planes de type treillis s'étendent au-delà de la surface non plane.

11. Implant selon l'une la revendication 1, dans lequel l'au moins quelques des entretoises de connexion externe (2905) définissent des treillis triangulaires possédant au moins un noeud à l'extrémité d'une entretoise polaire.

12. Implant selon l'une quelconque des revendications 1 à 11, dans lequel au moins une partie des entretoises couplées à un noeud au niveau de l'extrémité d'une entretoise polaire sont courbes pour former une surface extérieure arrondie de l'implant.

13. Implant selon l'une quelconque des revendications 1 à 10, dans lequel la pluralité d'unités planes de type treillis définit une pluralité d'unités planes de type treillis de surface de côté définissant une surface de côté extérieure de l'implant, les unités planes de type treillis de surface de côté comprenant une pluralité d'entretoises couplées à une pluralité de noeuds ; et dans lequel une pluralité d'entretoises de surface s'étend depuis un ou plusieurs des noeuds d'une ou de plusieurs unités planes de type treillis de surface de côté en direction de la partie interne de l'implant, dans lequel l'angle entre les entretoises de surface et les entretoises définissant les unités planes de type treillis de surface de côté est essentiellement supérieur à, ou essentiellement inférieure à 90 ; dans lequel l'implant comprend en outre une pluralité de noeuds internes couplant les entretoises surface les unes aux autres pour définir une surface externe de l'implant.
